# EUROPEAN PATENT SPECIFICATION

(11) **EP 2 803 340 B1**
(45) Date of publication and mention of the grant of the patent: **04.10.2017**
(21) Application number: 13736412.1
(22) Date of filing: 10.01.2013
(51) Int. Cl.: A61F 2/954, A61M 25/00, A61M 25/09, A61M 25/06, A61M 25/01, A61F 2/856, A61F 2/915, A61F 2/966

(54) **BIFURCATED STENT DELIVERY SYSTEM AND GUIDE CATHETER FOR SAME**
SYSTEM ZUR VERABREICHUNG GEGABELTER STENTS UND FÜHRUNGSKATHETER DAFÜR
SYSTÈME DE MISE EN PLACE D'ENDOPROTHÈSES À PLUSIEURS BRANCHES ET CATHÉTER-GUIDE À CET EFFET

(30) Priority: 12.01.2012 CN 201210008588
(43) Date of publication of application: 19.11.2014
(73) Proprietor: MicroPort Endovascular (Shanghai) Co., Ltd., Pudong New District Shanghai Shanghai 201318 (CN)
(72) Inventor: ZHU, Qing, Shanghai 201203 (CN); HUANG, Dingguo, Shanghai 201203 (CN); LUO, Qiyi, Shanghai 201203 (CN); GAO, Yanbin, Shanghai 201203 (CN)
(74) Representative: Dennemeyer & Associates S.A.
(86) International application number: PCT/CN2013/070323
(87) International publication number: WO 2013/104324

(56) References cited:
- WO-A1-2006/093550
- WO-A1-2006/093550
- WO-A1-2011/116308
- WO-A2-01/70299
- WO-A2-2005/112798
- WO-A2-2005/112798
- CN-A- 1 441 654
- CN-A- 101 897 629
- CN-A- 102 525 699
- US-A1- 2003 149 444
- US-A1- 2003 149 444
- US-A1- 2004 143 286
- US-A1- 2004 143 286

## Description

### TECHNICAL FIELD

The invention relates to a guide catheter of a branched stent delivery system which is useful for surgical treatment of aortic lesions according to the preamble of claim 1. The invention further relates to a branched stent delivery system comprising such guide catheter.

### BACKGROUND ART

In clinics, a substantial part of aortic aneurysms involve a number of branched arteries of the aorta. In a traditional treatment, for patients suffering from a Stanford A-type aortic dissection, i.e., an entire aortic lesion (including the ascending aorta, the aortic arch, the descending aorta and the abdominal aorta), a median thoracotomy operation is generally performed, in which an artificial blood vessel is used in place of the ascending aorta arch, and a piece of artificial blood vessel (commonly known as an elephant trunk) is delivered into the true lumen of the descending aorta, in order to prepare for a second operation or a second interventional treatment (Covered Stent Implantation). For most patients, the treatment cannot be completed in one operation, and the second operation needs to perform thoracotomy from below the left ribs. The second operation has a big risk, a high cost, and it is somewhat difficult in the connection between the operations. Such traditional prosthetic blood vessel replacement is extremely complex, causes a very large wound, and the death rate is extremely high. So it is necessary to perform the treatment using a minimally invasive surgery method.

An intraoperative stent has been developed in recent years, and it can effectively solve the above problem occurring due to the second operation, i.e., the entire treatment can be completed in one operation. In the operation, a covered stent is directly delivered into the true lumen of the descending aorta that cannot be exposed via the aorta that has been cut open, thereby achieving the object of treatment. The covering material on the intraoperative stent can be stitched with the artificial blood vessel used in the operation, thereby achieving a complete artificial vascular prosthesis replacement system and achieving the object of completing the entire treatment in one operation. The adoption of the intraoperative stent to perform the operation not only completes the entire treatment in one operation, but also shortens the operation time and relieves the pain suffered by a patient due to the second operation.

Chinese patent application for invention CN101332133A discloses a braiding device for a branched stent or implant and a manufacturing method for the same, in which a single fabric formed by braiding a plurality of filaments has a trunk portion and two legs hinged to each other. The legs include all of the plurality of filaments, and the trunk portion includes a subset of the same plurality of filaments. The manufacturing method comprises braiding the hinged legs on a mandrel, and keeping a wire ring between the two hinged legs to braid the trunk portion of the stent or implant.

However, in the current minimally invasive surgery of the thoracic aorta and a plurality of branched arteries, it is a difficulty how to safely and smoothly deliver respective stent branches of a multi-branch stent to the lesion locations within the respective branched blood vessel cavities so as to safely release the stent.

Chinese patent application for invention CN101897629A relates to a branched covered stent delivery system, which discloses delivery of a unilateral-branch or single-branch stent. This delivery system cannot solve the problem of the safe release during the delivery process of the multi-branch stent. In the case of the multi-branch stent, this system cannot ensure that the plurality of guide wires do not intertwine and interfere with each other, and cannot make the respective guide wires smoothly enter the respective branched blood vessel cavities.

WO 2011/116308 A1 discloses a stent graft delivery device comprising a stent delivery system for introducing a guide wire into a branched blood vessel cavity. Each guide wire channel is provided with an inlet and an outlet for the guide wire, whereby a guide catheter is axially split between the inlet and the outlet of the guide wire.

### SUMMARY OF THE INVENTION

In view of the above, the object of the invention is to solve the difficulty of safely and smoothly delivering a branched stent to the lesion locations of corresponding branched blood vessels in a minimally invasive surgery of the thoracic aorta and a plurality of branched arteries.

This object is solved by a guide catheter having the features of claim 1.

According to a first aspect of the invention, a guide catheter for a branched stent delivery system is provided, which guide catheter is used for guiding a guide wire into a corresponding branched blood vessel cavity in order that a stent branch of a branched stent is subsequently dragged into its respective branched blood vessel cavity. The guide catheter is provided with at least one guide wire channel extending axially for guiding the guide wire, each guide wire channel is provided with an inlet for the guide wire and a corresponding outlet for the guide wire, and the guide catheter is axially split between the inlet for the guide wire and the outlet for the guide wire of the respective guide wire channel to form an incision, via which the respective guide wire can be separated from the guide catheter. In this manner, the catheter can be separated from the guide wire with the catheter being not withdrawn at both of proximal and distal ends thereof.

Generally, the branched stent has three stent branches for three branched arteries of the aorta. Thus, the guide catheter has three guide wire channels, each of the channels guiding one guide wire.

The guide catheter is made of a polymer composite material or a metallic material, or is a pipe fitting in the form of a composite structure. The guide catheter can be constructed as a double-layered or multi-layered pipe fitting, which includes an inner pipe fitting and an outer pipe fitting, both of the inner and outer pipe fittings being axially split between the inlet for the guide wire and the outlet for the guide wire of the respective guide wire channel to form the incision. The guide catheter can also be constructed as a single pipe fitting. According to the invention, the incision is in the form of a line, a tearing line or a groove.

According to the invention the guide catheter is a pipe fitting of combined split portions, the incision is defined at the junction between the split portions.

Preferably, the guide catheter is provided with a radiopaque mark at the respective guide wire outlet. This can facilitate accurate positioning of the guide catheter during the operation.

According to the invention, the guide catheter comprises a control guide wire channel extending axially for guiding a control guide wire. The guide catheter is also axially split on an extension of the control guide wire channel to form an incision, via which the control guide wire can be separated from the guide catheter. The incision can be in the form of a line, a tearing line or a groove.

The guide catheter is a pipe fitting of combined split portions and an incision is further defined at the junction of the split portions, via which the control guide wire can pass so as to be separated from the guide catheter.

According to a second aspect of the invention, a branched stent delivery system is provided, which system comprises the above-mentioned guide catheter.

According to the invention, the branched stent delivery system further comprises a manipulating handle, a sheath, a branched stent, a control guide wire for introducing and releasing the branched stent and a control switch, the branched stent being connected with the control guide wire and comprising at least one stent branch, and the system comprising a guide wire for dragging the stent branch, wherein the manipulating handle and the control switch are controlled in such a way that firstly, a guide catheter is introduced till the respective guide wire outlet is aligned with a corresponding branched blood vessel; after that, the guide wire is guided into a corresponding branched blood vessel cavity via the guide catheter; then the guide catheter is separated from the guide wire with the guide wire being kept in the branched blood vessel cavity; after that, the branched stent is introduced by means of the control guide wire in such a way that the stent branch is dragged into its respective branched blood vessel cavity via the guide wire; finally, the branched stent is released by means of the control guide wire, and the guide wire and the control guide wire are withdrawn.

According to the invention, the guide catheter can be withdrawn after being separated from the guide wire, and the control guide wire can be independently introduced via another catheter.

Preferably, the manipulating handle and the control switch can be controlled in such a way that both of the guide wire and the control guide wire are guided into the respective aorta blood vessel cavity and branched artery blood vessel cavity via the guide catheter, and then the guide catheter is separated from the guide wire and the control guide wire with the guide wire and the control guide wire being kept in the respective blood vessel cavities.

According to the invention, the control guide wire and the guide wire can be introduced simultaneously or not.

The guide catheter and the stent delivery system according to the invention can ensure that the guide wires do not intertwine and interfere with each other during the delivery process, and can ensure that the respective guide wires smoothly and safely enter the respective branched blood vessel cavities. Further, according to the invention, the guide wire is firstly introduced into the branched blood vessel cavity via the guide catheter, thereby creating a passage for dragging the stent branch. In this manner, the stent branch can be delivered safely and smoothly to its respective branched blood vessel cavity along the created passage.

### BRIEF DESCRIPTION OF THE DRAWINGS

Fig. 1 is a schematic diagram of the construction of a stent delivery system and a guide catheter;
Figs. 1A-1B are partial enlarged views of a guide catheter;
Figs. 2-2A are a schematic diagram and a partial enlarged view of a guide catheter in the form of a single pipe fitting;
Figs. 3-3A are a schematic diagram and a partial enlarged view of a guide catheter in the form of a double-layered pipe fitting;
Figs. 4A-4C are schematic views of incision types for a guide catheter;
Figs. 5A-5C are schematic views of incision types with the control guide wire channel also being provided with a incision;
Fig. 6 is a schematic diagram of a guide catheter according to the present invention in the form of a pipe fitting of combined split portions;
Fig. 7 is a schematic diagram of introducing a guide wire into a branched blood vessel cavity via a guide catheter;
Fig. 8 is a schematic diagram of dragging a stent branch into a branched blood vessel cavity via a guide wire;
Fig. 9 is a schematic diagram after the release of a stent.

List of reference numbers
1 : Manipulating handle
2 : Control guide wire
3 : Control switch
4 : Sheath
5, 6, 7 : Guide wire
8, 8' : Guide catheter
9 : Branched stent
10, 11, 12 : Stent branch
20, 20' : Tapered head of the guide catheter
30 : Guide wire withdrawal incision
100, 200 : Incision in the form of a line
100', 200' : Incision in the form of tearing line
100', 200' : Incision in the form of a groove
a, b, c : Inlet for the guide wire
d, e, f : Outlet for the guide wire

### DETAILED DESCRIPTION

Detailed descriptions of the specific embodiments of the invention are given below with reference to the figures.

In the embodiments below, the branched stent has three stent branches for three branched arteries. Thus, the guide catheter has three guide wire channels, each of the channels guiding one guide wire, so as to drag the three stent branches, respectively. However, it is apparent to those skilled in the art that the number of the guide wire channels can be varied depending on the number of the stent branches. In the case that there is only one stent branch or two stent branches, there may be only one or two guide wire channels.

Further, in the embodiments below, the guide catheter has a control guide wire cavity. However, it is apparent to those skilled in the art that the control guide wire can be guided into the aorta blood vessel cavity via another catheter. Further, the control guide wire and the guide wire can be introduced simultaneously or not.

In addition, for the sake of description, the terms "distal end" and "proximal end" are used herein. An end proximate to an operating end (i.e., a manipulating handle 1) is referred to as a proximal end, and an end away from the manipulating handle 1 is referred to as a distal end.

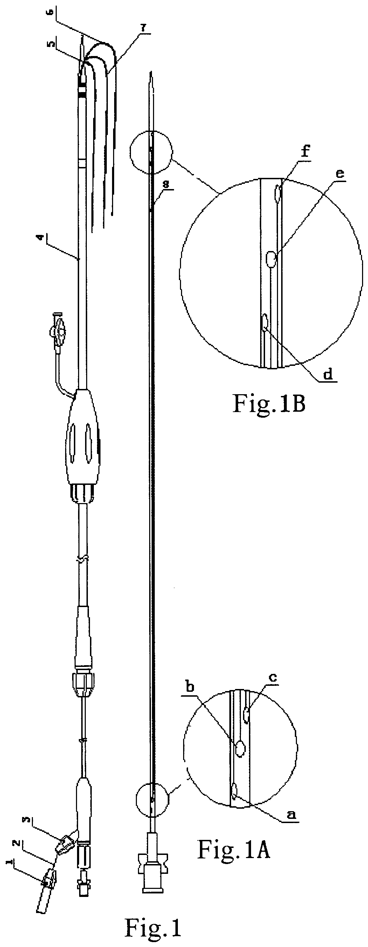

As shown in Fig. 1 , the stent delivery system of the invention comprises a manipulating handle 1, a control guide wire 2, a control switch 3, a sheath 4, guide wires 5, 6, 7 and a guide catheter 8. The sheath 4 is made of a flexible polymer material such as PET and PTFE. A releasable branched stent 9 is entirely or semi-entirely enclosed by the sheath 4. As an alternative, the sheath 4 can be constructed as a multi-layered sheath, as disclosed in Chinese patent application for Invention CN101897629A in the name of the applicant.

The stent 9 has three stent braches 10, 11, 12, the stent body and the stent branches are connected with the control guide wire 2 and the guide wires 5, 6, 7, respectively, so as to be dragged, and both of the stent branches and the stent body are released by operating the control guide wire 2. The respective guide wires can be actuated via the manipulating handle 1 and the control switch 3. The above contents are well known to those skilled in the art.

Now turn to Figs. 1A-1B. As shown in the figures, the guide catheter 8 is provided at its proximal end with three inlets a, b, c, for the guide wire, and is provided at its distal end with three outlets d, e, f. for the guide wire. The three guide wires 5, 6, 7 can be introduced from the inlets and extend out from the outlets. The outlets are further provided with a radiopaque mark (not shown) to facilitate accurate positioning of the catheter. The guide catheter 8 is made of a polymer composite material or a metallic material, or is constructed as a pipe fitting in the form of a composite structure.

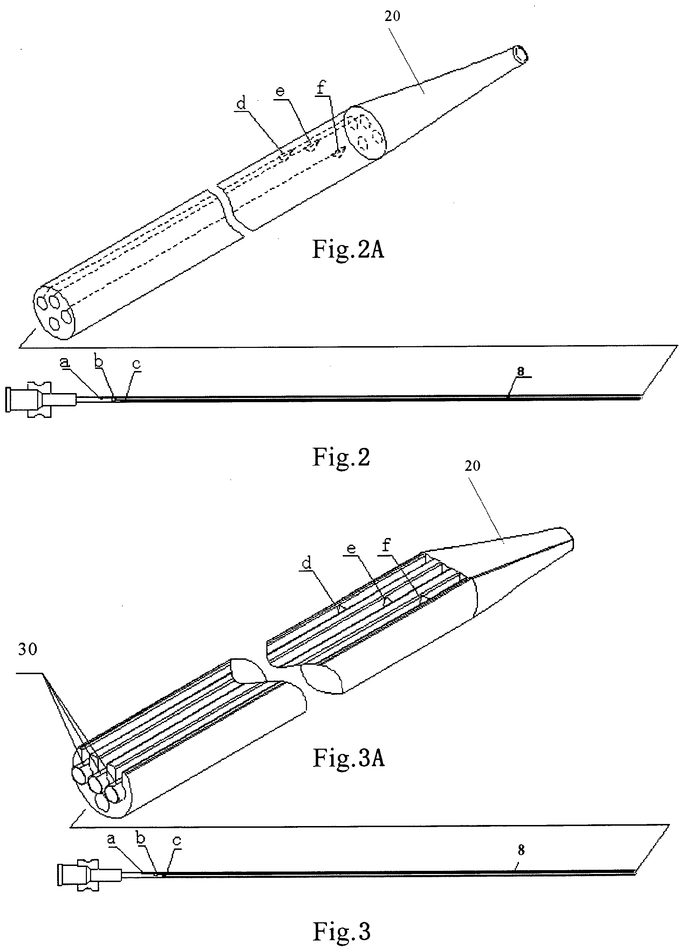

As shown in Figs. 2-2A , the guide catheter 8 is provided at its distal end with a tapered head 20 to facilitate the introduction of the catheter into the aorta blood vessel, and is axially split between the inlet for the guide wire and the outlets for the guide wire of the respective guide wire channels to form incisions. The guide catheter 8 is further provided with a control guide wire channel (not indicated) for guiding the control guide wire 2. In this case, the guide catheter 8 is a single pipe fitting. As an alternative, the guide catheter 8 can be a double-layered pipe fitting, as shown in Figs. 3-3A , in which both of the inner and outer pipe fittings are axially split between the inlets for the guide wires and the outlets for the guide wires of the respective guide wire channels to form incisions. Guide wire withdrawal incisions 30 are indicated in Fig. 3A.

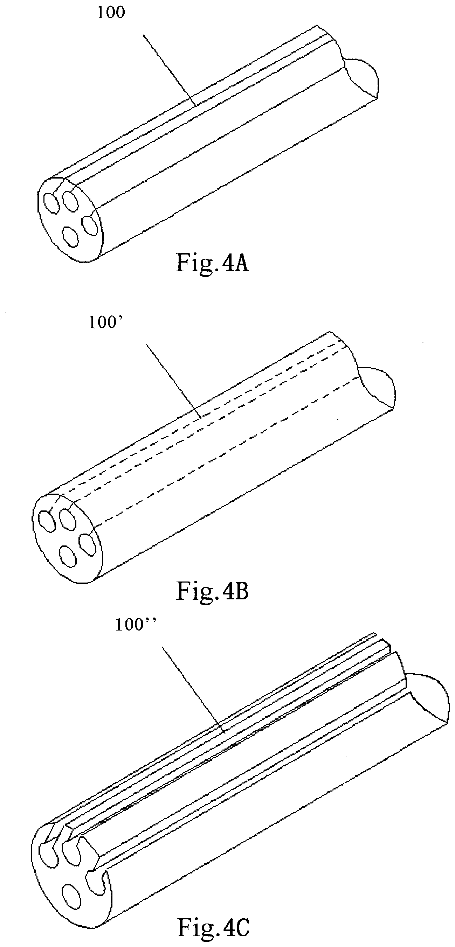

The incisions can be incisions in the form of a line 100 as shown in Fig. 4A , in the form of a tearing line 100' as shown in Fig. 4B , or in the form of a groove 100" as shown in Fig. 4C . The incisions in the form of a tearing line refer to a series of intermittent tearing lines axially formed along the guide catheter 8. When the guide wire is separated from the guide catheter, a through incision can be easily formed by breaking the tearing lines.

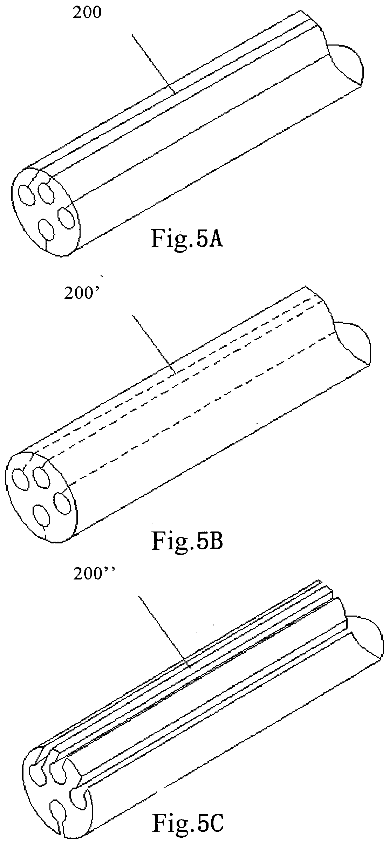

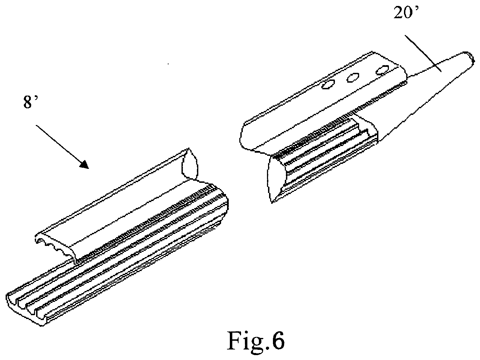

The guide catheter 8 can be axially split on an extension of the control guide wire channel to form an incision, as shown in Figs. 5A-5C . The incision can be also an incision in the form of a line 200, in the form of a tearing line 200' or in the form of a groove 200'. The guide catheter is constructed as a pipe fitting 8' of combined split portions, as shown in Fig. 6 , and the incision is formed at the junction of the split portions of the pipe fitting. The pipe fitting 8' also has a tapered head 20'.

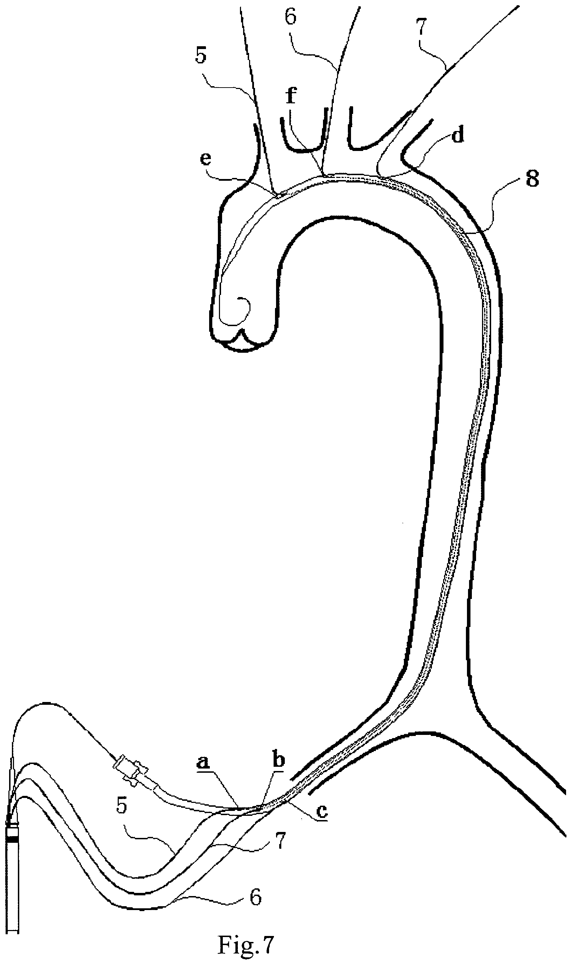

The descriptions of the operating method of the branched stent delivery system of the invention are given as follows with reference to Figs. 7-9 . Firstly, the guide catheter 8 is introduced from the iliac artery of the human body till the respective guide wire outlets d, e, f of the guide catheter 8 are aligned with the corresponding branched blood vessels, respectively. A radiopaque mark may be provided at the guide wire outlets to facilitate accurate positioning of the catheter.

Then, the guide wires 5, 6, 7 are introduced from the respective guide wire inlets a, b, c of the guide catheter 8, respectively, till the guide wires 5, 6, 7 extend out from the guide wire outlets d, e, f, as shown in Fig. 1 . Further, the guide wires 5, 6, 7 are introduced into the respective branched blood vessel cavities, respectively, as shown in Fig. 7 . The control guide wire 2 can be introduced simultaneously along with the guide wires via the guide catheter 8.

Then, while the guide wires 5, 6, 7 are kept in the respective branched blood vessel cavities, the guide wires 5, 6, 7 are separated from the guide catheter 8 via the respective incisions so as to withdraw the guide catheter 8 from the human body. Thus, the guide wires reach the desired blood vessel positions without intertwining and interfering with each other. The control guide wire 2 can be also separated from the guide catheter 8 via the respective incision.

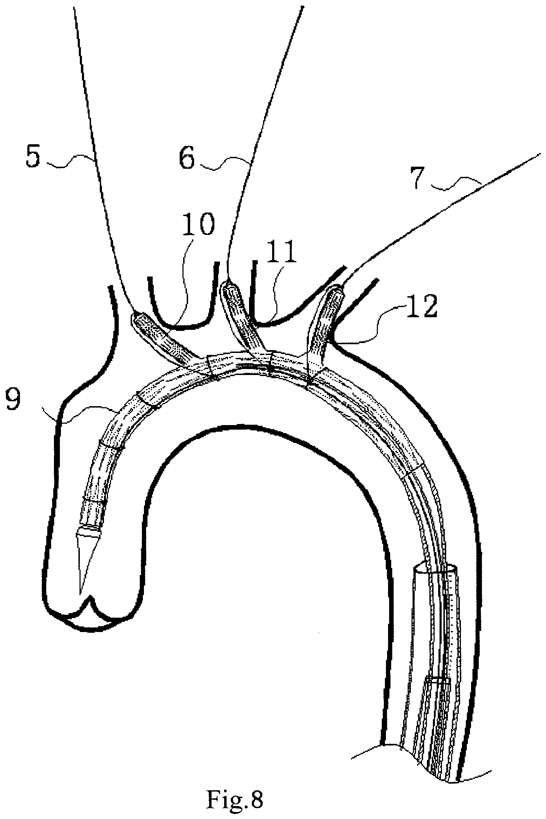

Then, the branched stent 9 is delivered in place by means of the catheter of the branched stent and the control guide wire 2, wherein the stent branches 10, 11, 12 are dragged into the respective branched blood vessel cavities by means of the guide wires 5, 6, 7, as shown in Fig. 8.

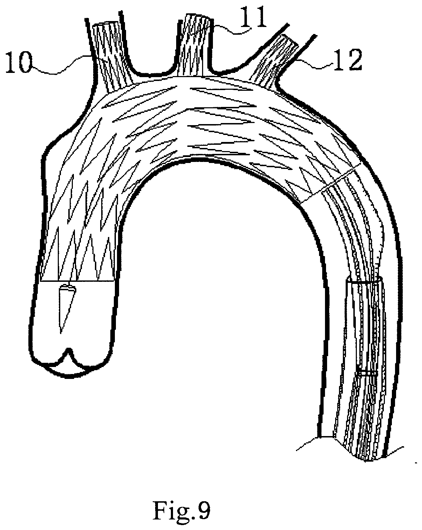

Thereafter, the branched stent 9 is released by means of the control guide wire 2, and then the respective guide wires 5, 6, 7 and the control guide wire 2 are withdrawn, as shown in Fig. 9. The guide wires can extend out from the aorta branched blood vessels and be distally withdrawn. Finally, the sheath 4 is withdrawn from the human body, thereby completing the stent implantation operation.

It is ensured by the guide catheter 8 of the invention that the plurality of guide wires 5, 6, 7 do not intertwine and interfere with each other during the delivery process, and can ensure that the guide wires enter the respective branched blood vessel cavities. Further, the stent branches are introduced along the passages created by the guide wires, so that the branched stent 9 is safely and smoothly delivered to the lesion locations of the aorta blood vessel and the plurality of branched artery blood vessels, and the stent 9 is safely released.

The guide catheter and the delivery system provided by the invention are not only suitable for delivery of a branched stent for the aorta but also suitable for delivery of a branched stent for other blood vessels.

The invention is definitely not limited to the specific disclosure mentioned in the above specific embodiments. Those skilled in the art can think of other variations, modifications, and additions without departing from the scope of the invention according to the claims.

## Claims

1. A guide catheter (8) for a branched stent delivery system, which is used for introducing a guide wire (5, 6, 7) into a corresponding branched blood vessel cavity in order that a stent branch (10, 11, 12) of a branched stent (9) is subsequently dragged into its corresponding branched blood vessel cavity, the guide catheter (8) is provided with at least one guide wire channel extending axially for guiding the guide wire (5, 6, 7), each guide wire channel is provided with an inlet (a, b, c) and a corresponding outlet (d, e, f) for the guide wire, the guide catheter (8) is axially split between the inlet for the guide wire and the outlet for the guide wire of the respective guide wire channel to form an incision, via which the respective guide wire (5, 6, 7) can be separated from the guide catheter (8), the guide catheter (8) further comprises a control guide wire channel extending axially for guiding a control guide wire (2), wherein the guide catheter (8) is axially split on an extension of the control guide wire channel to form another incision, via which the control guide wire (2) can be separated from the guide catheter (8),
**characterized in that** the guide catheter (8) is a pipe fitting (8') combining split portions, with the incision being defined at the junction between the split portions, and
wherein the guide catheter (8) further comprises a control guide wire cavity extending axially for guiding a control guide wire (2), and wherein said another incision is further defined at the junction of the split portions, via which the control guide wire (2) can pass so as to be separated from the guide catheter (8).

2. The guide catheter (8) of claim 1, wherein the guide catheter (8) is constructed as a double-layered or multi-layered pipe fitting, which includes an inner pipe fitting and an outer pipe fitting, both of the inner and outer pipe fittings being axially split between the inlet for the guide wire and the outlet for the guide wire of the respective guide wire channel to form the incision.

3. The guide catheter (8) of claim 1, wherein the guide catheter (8) is constructed as a single pipe fitting.

4. The guide catheter (8) of any one of claims 1-3, wherein the incision is in the form of a line (100; 200), a tearing line (100'; 200') or a groove (100"; 200").

5. The guide catheter (8) of any one of claims 1-3, wherein the guide catheter (8) is provided with a radiopaque mark at the respective outlet for guide wire (d, e, f).

6. The guide catheter (8) of claim 1, wherein each incision is in the form of a line (200), a tearing line (200') or a groove (200").

7. A branched stent delivery system, **characterized in that** the branched stent delivery system comprises a guide catheter (8) according to any one of claims 1-6.

8. The branched stent delivery system of claim 7, further comprising a manipulating handle (1), a sheath (4), a branched stent (9), a control guide wire (2) for introducing and releasing the branched stent (9) and a control switch (3), the branched stent (9) being connected with the control guide wire (2) and comprising at least one stent branch (10, 11, 12), and the system comprising a guide wire (5, 6, 7) for dragging the stent branch (10, 11, 12), wherein the manipulating handle (1) and the control switch (3) are controlled in such a way that the guide catheter (8) is firstly introduced till the respective outlet for guide wire is aligned with a corresponding branched blood vessel; after that, the guide wire (5, 6, 7) is guided into a corresponding branched blood vessel cavity via the guide catheter (8); then the guide catheter (8) is separated from the guide wire (5, 6, 7) with the guide wire being kept in the branched blood vessel cavity; after that, the branched stent (9) is introduced by means of the control guide wire (2) in such a way that the stent branch (10, 11, 12) is dragged into the respective branched blood vessel cavity via the guide wire (5, 6, 7); finally, the branched stent (9) is released by means of the control guide wire (2), and the guide wire (5, 6, 7) and the control guide wire (2) are withdrawn.

9. The branched stent delivery system of claim 8, wherein the manipulating handle (1) and the control switch (3) are controlled in such a way that both of the guide wire (5, 6, 7) and the control guide wire (2) are guided into the respective blood vessel cavity via the guide catheter (8), and then the guide catheter (8) is separated from the guide wire (5, 6, 7) and the control guide wire (2) with the guide wire and the control guide wire being kept in the blood vessel cavity.

## Patentansprüche

1. Führungskatheter (8) für ein verzweigtes Stentapplikationssystem, der zum Einführen eines Führungsdrahts (5, 6, 7) in einen entsprechenden verzweigten Blutgefäßhohlraum verwendet wird, damit ein Stentzweig (10, 11, 12) eines verzweigten Stents (9) anschließend in seinen entsprechenden verzweigten Blutgefäßhohlraum gezogen wird,
wobei der Führungskatheter (8) mit mindestens einem axial verlaufenden Führungsdrahtkanal zum Führen des Führungsdrahts (5, 6, 7) versehen ist, jeder Führungsdrahtkanal mit einem Einlass (a, b, c) und einem entsprechenden Auslass (d, e, f) für den Führungsdraht versehen ist, der Führungskatheter (8) zwischen dem Einlass für den Führungsdraht und dem Auslass für den Führungsdraht des jeweiligen Führungsdrahtkanals axial geteilt ist, um einen Einschnitt zu bilden, durch welchen der jeweilige Führungsdraht (5, 6, 7) vom Führungskatheter (8) getrennt werden kann,
der Führungskatheter (8) ferner einen axial verlaufenden Steuerführungsdrahtkanal zum Führen eines Steuerführungsdrahts (2) umfasst, wobei der Führungskatheter (8) auf einer Ausdehnung des Steuerführungsdrahtkanals axial geteilt ist, um einen anderen Einschnitt zu bilden, durch welchen der Steuerführungsdraht (2) vom Führungskatheter (8) getrennt werden kann,
**dadurch gekennzeichnet, dass** der Führungskatheter (8) ein Rohrformstück (8') ist, das geteilte Teile kombiniert, wobei der Einschnitt am Übergang zwischen den geteilten Teilen definiert ist, und
wobei der Führungskatheter (8) ferner einen axial verlaufenden Steuerführungsdrahthohlraum zum Führen eines Steuerführungsdrahts (2) umfasst, und wobei der andere Einschnitt ferner am Übergang der geteilten Teile definiert ist, durch welchen der Steuerführungsdraht (2) durchtreten kann, um vom Führungskatheter (8) getrennt zu werden.

2. Führungskatheter (8) nach Anspruch 1, wobei der Führungskatheter (8) als zweischichtiges oder mehrschichtiges Rohrformstück aufgebaut ist, das ein inneres Rohrformstück und ein äußeres Rohrformstück umfasst, wobei sowohl das innere als auch das äußere Rohrformstück zwischen dem Einlass für den Führungsdraht und dem Auslass für den Führungsdraht des jeweiligen Führungsdrahtkanals axial geteilt sind, um den Einschnitt zu bilden.

3. Führungskatheter (8) nach Anspruch 1, wobei der Führungskatheter (8) als ein einziges Rohrformstück aufgebaut ist.

4. Führungskatheter (8) nach einem der Ansprüche 1 bis 3, wobei der Einschnitt in Form einer Linie (100; 200), einer Reißlinie (100'; 200') oder einer Nut (100"; 200") ist.

5. Führungskatheter (8) nach einem der Ansprüche 1 bis 3, wobei der Führungskatheter (8) am jeweiligen Auslass (d, e, f) für Führungsdraht mit einer röntgenstrahlenundurchlässigen Markierung versehen ist.

6. Führungskatheter (8) nach Anspruch 1, wobei jeder Einschnitt in Form einer Linie (200), einer Reißlinie (200') oder einer Nut (200") ist.

7. Verzweigtes Stentapplikationssystem, **dadurch gekennzeichnet, dass** das verzweigte Stent-Applikationssystem einen Führungskatheter (8) nach einem der Ansprüche 1 bis 6 umfasst.

8. Verzweigtes Stentapplikationssystem nach Anspruch 7, ferner umfassend einen Bediengriff (1), eine Hülse (4), einen verzweigten Stent (9), einen Steuerführungsdraht (2) zum Einführen und Freigeben des verzweigten Stents (9) und einen Steuerschalter (3), wobei der verzweigte Stent (9) mit dem Steuerführungsdraht (2) verbunden ist und mindestens einen Stentzweig (10, 11, 12) umfasst, und das System einen Führungsdraht (5, 6, 7) zum Ziehen des Stentzweigs (10, 11, 12) umfasst, wobei der Bediengriff (1) und der Steuerschalter (3) derart gesteuert werden, dass zunächst der Führungskatheter (8) eingeführt wird, bis der jeweilige Auslass für Führungsdraht mit einem entsprechenden verzweigten Blutgefäß ausgerichtet ist; danach der Führungsdraht (5, 6, 7) durch den Führungskatheter (8) in einen entsprechenden verzweigten Blutgefäßhohlraum geführt wird; dann der Führungskatheter (8) vom Führungsdraht (5, 6, 7) getrennt wird, wobei der Führungsdraht im verzweigten Blutgefäßhohlraum gehalten wird; danach der Stentzweig (9) mittels des Steuerführungsdrahts (2) derart eingeführt wird, dass der Stentzweig (10, 11, 12) durch den Führungsdraht (5, 6, 7) in den jeweiligen verzweigten Blutgefäßhohlraum gezogen wird; schließlich der Stentzweig (9) mittels des Steuerführungsdrahts (2) freigegeben wird, und der Führungsdraht (5, 6, 7) und der Steuerführungsdraht (2) zurückgezogen werden.

9. Verzweigtes Stentapplikationssystem nach Anspruch 8, wobei der Bediengriff (1) und der Steuerschalter (3) derart gesteuert werden, dass sowohl der Führungsdraht (5, 6, 7) als auch der Steuerführungsdraht (2) durch den Führungskatheter (8) in den jeweiligen Blutgefäßhohlraum geführt werden, und dann der Führungskatheter (8) vom Führungsdraht (5, 6, 7) und vom Steuerführungsdraht (2) getrennt wird, wobei der Führungsdraht und der Steuerführungsdraht im Blutgefäßhohlraum gehalten werden.

## Revendications

1. Cathéter-guide (8) pour un système de pose de stent à plusieurs branches, qui est utilisé pour introduire un fil-guide (5, 6, 7) dans une cavité de vaisseau sanguin à plusieurs branches correspondante de sorte qu'une branche de stent (10, 11, 12) d'un stent à plusieurs branches (9) est sensiblement entraînée dans sa cavité de vaisseau sanguin à plusieurs branches correspondante, le cathéter-guide (8) est prévu avec au moins un canal de fil-guide s'étendant axialement pour guider le fil-guide (5, 6, 7), chaque canal de fil-guide est prévu avec une entrée (a, b, c) et une sortie (d, e, f) correspondante pour le fil-guide, le cathéter-guide (8) est axialement partagé entre l'entrée pour le fil-guide et la sortie pour le fil-guide du canal de fil-guide respectif afin de former une incision, via laquelle le fil-guide (5, 6, 7) respectif peut être séparé du cathéter-guide (8),
le cathéter-guide (8) comprend en outre un canal de fil-guide de commande s'étendant axialement pour guider un fil-guide de commande (2), dans lequel le cathéter-guide (8) est axialement partagé sur une extension du canal de fil-guide de commande pour former une autre incision via laquelle le fil-guide de commande (2) peut être séparé du cathéter-guide (8),
**caractérisé en ce que** le cathéter-guide (8) est un raccord de tuyau (8') combinant des parties partagées, avec l'incision qui est définie au niveau de la jonction entre les parties partagées, et
dans lequel le cathéter-guide (8) comprend en outre une cavité de fil-guide de commande s'étendant axialement pour guider un fil-guide de commande (2), et dans lequel ladite autre incision est en outre définie au niveau de la jonction des parties partagées, via lesquelles le fil-guide de commande (2) peut passer afin d'être séparé du cathéter-guide (8).

2. Cathéter-guide (8) selon la revendication 1, dans lequel le cathéter-guide (8) est fabriqué sous la forme d'un raccord de tuyau à deux couches ou plusieurs couches, qui comprend un raccord de tuyau interne et un raccord de tuyau externe, à la fois les raccords de tuyau interne et externe étant axialement séparés entre l'entrée pour le fil-guide et la sortie pour le fil-guide du canal de fil-guide respectif afin de former l'incision.

3. Cathéter-guide (8) selon la revendication 1, dans lequel le cathéter-guide (8) est fabriqué comme un seul raccord de tuyau.

4. Cathéter-guide (8) selon l'une quelconque des revendications 1 à 3, dans lequel l'incision se présente sous la forme d'une ligne (100 ; 200), d'une ligne de déchirure (100' ; 200') ou d'une rainure (100" ; 200'').

5. Cathéter-guide (8) selon l'une quelconque des revendications 1 à 3, dans lequel le cathéter-guide (8) est prévu avec une marque radio-opaque au niveau de la sortie respective pour le fil-guide (d, e, f).

6. Cathéter-guide (8) selon la revendication 1, dans lequel chaque incision se présente sous la forme d'une ligne (200), d'une ligne de déchirure (200') ou d'une rainure (200'').

7. Système de pose de stent à plusieurs branches, **caractérisé en ce que** le système de pose de stent à plusieurs branches comprend un cathéter-guide (8) selon l'une quelconque des revendications 1 à 6.

8. Système de pose de stent à plusieurs branches selon la revendication 7, comprenant en outre une poignée de manipulation (1), une gaine (4), un stent à plusieurs branches (9), un fil-guide de commande (2) pour introduire et libérer le stent à plusieurs branches (9) et un commutateur de commande (3), le stent à plusieurs branches (9) étant raccordé avec le fil-guide de commande (2) et comprenant au moins une branche de stent (10, 11, 12) et le système comprenant un fil-guide (5, 6, 7) pour entraîner la branche de stent (10, 11, 12), dans lequel la poignée de manipulation (1) et le commutateur de commande (3) sont commandés de sorte que le cathéter-guide (8) est tout d'abord introduit jusqu'à ce que la sortie respective pour le fil-guide soit alignée avec un vaisseau sanguin à plusieurs branches correspondant ; après cela, le fil-guide (5, 6, 7) est guidé dans une cavité de vaisseau sanguin à plusieurs branches correspondante via le cathéter-guide (8) ; ensuite le cathéter-guide (8) est séparé du fil-guide (5, 6, 7) avec le fil-guide qui est maintenu dans la cavité de vaisseau sanguin à plusieurs branches ; après cela, le stent à plusieurs branches (9) est introduit au moyen du fil-guide de commande (2) de sorte que la branche de stent (10, 11, 12) est entraînée dans la cavité de vaisseau sanguin à plusieurs branches respective via le fil-guide (5, 6, 7) ; finalement, le stent à plusieurs branches (9) est libéré au moyen du fil-guide de commande (2), et le fil-guide (5, 6, 7) et le fil-guide de commande (2) sont retirés.

9. Système de pose de stent à plusieurs branches selon la revendication 8, dans lequel la poignée de manipulation (1) et le commutateur de commande (3) sont commandés de sorte que le fil-guide (5, 6, 7) et le fil-guide de commande (2) sont tous deux guidés dans la cavité de vaisseau sanguin respective via le cathéter-guide (8), et ensuite le cathéter-guide (8) est séparé du fil-guide (5, 6, 7) et du fil-guide de commande (2) avec le fil-guide et le fil-guide de commande étant maintenu dans la cavité de vaisseau sanguin.
